# EUROPÄISCHE PATENTSCHRIFT

(11) **EP 4 282 348 B1**
(45) Veröffentlichungstag und Bekanntmachung des Hinweises auf die Patenterteilung: **13.08.2025**
(21) Anmeldenummer: 23174540.7
(22) Anmeldetag: 22.05.2023
(51) Int. Cl.: A61B 17/15, A61B 17/17

(54) **CHIRURGISCHES INSTRUMENT**
SURGICAL INSTRUMENT
INSTRUMENT CHIRURGICAL

(30) Priorität: 24.05.2022 DE 102022205172
(43) Veröffentlichungstag der Anmeldung: 29.11.2023
(73) Patentinhaber: Aesculap AG, 78532 Tuttlingen (DE)
(72) Erfinder: Anhorn, Svenja, 72535 Heroldstatt (DE); Firmbach, Franz-Peter, 78576 Emmingen-Liptingen (DE)
(74) Vertreter: Patentanwälte Ruff, Wilhelm, Beier, Dauster & Partner mbB

(56) Entgegenhaltungen:
- EP-A1- 4 245 229
- US-A1- 2015 045 801
- US-A1- 2021 236 143

## Beschreibung

Die Erfindung betrifft ein chirurgisches Instrument mit den oberbegrifflichen Merkmalen des Anspruchs 1.

Die Verwendung von orthopädischen Prothesen als künstlicher Ersatz für beschädigte oder abgenutzte natürliche Knochenstrukturen ist gängige medizinische Praxis. Insbesondere Hüft- oder Kniegelenkersatzoperationen gehören mittlerweile zum Standardrepertoire der chirurgischen Orthopädie.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch eine Kniegelenkprothese ersetzt. Solche Kniegelenkprothesen umfassen üblicherweise eine Femurkomponente, welche am distalen Ende des Femurs implantiert wird, und eine Tibiakomponente, welche am proximalen Ende der Tibia implantiert wird. Um eine einwandfreie Funktion des künstlichen Gelenkersatzes zu gewährleisten, müssen die besagten Komponenten hinsichtlich ihrer Lage und Orientierung in Bezug auf die Anatomie des Patienten und dessen Körperachsen in definierter Weise möglichst präzise positioniert werden. Andernfalls ist mit einem für den Patienten nicht zufriedenstellenden Ergebnis zu rechnen. Hinsichtlich der Positionierung der Komponenten existieren unterschiedliche chirurgische Ansätze.

Ein als Mechanical Alignment bekannter und bislang vorwiegend verwendeter Ansatz sieht vor, dass die Position und Ausrichtung der künstlichen Gelenkachsen der Kniegelenkprothese mechanisch ideal und insoweit ohne Berücksichtigung etwaiger orthopädischer Fehlstellungen des Patienten festgelegt werden. Hierbei dient oftmals die Längsachse der Tibia als Referenzachse für die Ausrichtung und Positionierung. Klinische Studien haben gezeigt, dass der Mechanical Alignment-Ansatz zu einer als unnatürlich empfundenen Funktion des künstlichen Kniegelenks führen kann.

Als ein weiterer Ansatz ist das sogenannte Kinematic Alignment (nachfolgend auch als KA abgekürzt) bekannt. Bei dieser Vorgehensweise werden die Femurkomponente und die Tibiakomponente unter Berücksichtigung etwaiger orthopädischer Fehlstellungen des Patienten positioniert. Ziel hierbei ist es, die natürliche und unter Umständen mit Fehlstellungen behaftete Gelenkausrichtung des Patienten wiederherzustellen. Klinische Studien haben gezeigt, dass der Kinematic Alignment-Ansatz oftmals mit einer verbesserten Patientenzufriedenheit einhergeht. Insbesondere die Funktion des künstlichen Kniegelenks wird seitens der Patienten als eher natürlich empfunden.

Mit dem Bestreben, die Patientenzufriedenheit weiter zu verbessern, geht ein grundsätzlicher Bedarf nach möglichst präzisen, einfach verwendbaren und kosteneffizienten chirurgischen Instrumenten zur Umsetzung des KA einher. Die vorliegende Erfindung befasst sich mit einem solchen chirurgischen Instrument, im Speziellen mit einem chirurgischen Instrument zur Ermittlung einer Größe und zur Einstellung einer Innen-/Außenrotation (interne/externe Rotation, abgekürzt I/E-Rotation) der Femurkomponente. Solche Instrumente werden auch als femoral sizer oder femoral sizing system bezeichnet. Aus dem Stand der Technik sind unterschiedliche femoral sizer bekannt.

Aus der US 2021/236143 A1 ist ein chirurgisches Instrument mit den oberbegrifflichen Merkmalen des Anspruchs 1 bekannt. Aus der US 2015/045801 A1 und der EP 4 245 229 A1 sind weitere chirurgische Instrumente mit jeweils einem Referenzblock, einem Taster, mediolateral beabstandeten Referenzfüßen, wenigstens zwei Referenzbohrungen und wenigstens einem Kompensationselement bekannt.

Aufgabe der Erfindung ist es, ein chirurgisches Instrument bereitzustellen, das Vorteile gegenüber dem Stand der Technik bietet und insbesondere eine verbesserte Einstellung der I/E-Rotation und/oder Größenermittlung ermöglicht.

Diese Aufgabe wird durch das Bereitstellen eines chirurgischen Instruments mit den Merkmalen des Anspruchs 1 gelöst.

Das erfindungsgemäße chirurgische Instrument weist auf: einen Referenzblock mit einer proximal orientierten Blockrückseite, welche zur Anlage an einer distalen Stirnfläche eines resezierten Femurs eingerichtet ist, einen Taster, welcher im Bereich einer anterioren Blockoberseite des Referenzblocks an demselben angeordnet ist und eine Tasterspitze aufweist, welche proximal über die Blockrückseite ragt und zur Anlage an einer anterioren Fläche des Femurs eingerichtet ist, zwei mediolateral voneinander beabstandete Referenzfüße, welche jeweils im Bereich einer der Blockoberseite posterior gegenüberliegenden Blockunterseite des Referenzblocks an demselben angeordnet sind und jeweils eine anterior orientierte sowie proximal über die Blockrückseite ragende Fußoberseite aufweisen, wobei die Fußoberseiten in einer gemeinsamen Referenzebene angeordnet sind und jeweils zur Anlage an einer posterioren Kondyle des Femurs eingerichtet sind, wenigstens zwei Referenzbohrungen, welche jeweils orthogonal zu der Blockrückseite durchgängig von derselben bis auf eine distal gegenüberliegende Blockvorderseite erstreckt und jeweils zur Aufnahme eines in die distale Stirnfläche des Femurs einbringbaren Referenzpins eingerichtet sind, wobei die wenigstens zwei Referenzbohrungen entlang einer virtuellen Abstandslinie, welche mediolateral und parallel zu der Referenzebene längserstreckt ist, voneinander beabstandet sind, wobei wenigstens ein Kompensationselement vorhanden und zur form- und/oder kraftschlüssigen lösbaren Verbindung mit einem der beiden Referenzfüße eingerichtet ist, und wobei das wenigstens eine Kompensationselement eine anterior orientierte Elementoberseite aufweist, welche anstelle der betreffenden Fußoberseite zur Anlage an der betreffenden posterioren Kondyle eingerichtet ist. Die erfindungsgemäße Lösung geht von der Überlegung aus, dass die Einstellung der I/E-Rotation und/oder die Größenermittlung im Rahmen des KA idealerweise ausgehend von der ursprünglichen posterioren Kondylenlinie des Femurs erfolgen sollte. Dabei meint ursprünglich in diesem Zusammenhang ohne kondyläre Defekte, wie beispielsweise Knorpelabnutzungen, Unfallschäden oder dergleichen. Liegen kondyläre Defekte vor, weicht die posteriore Kondylenlinie folglich von ihrer ursprünglichen Ausrichtung ab. Eine Einstellung der I/E-Rotation und/oder Größenermittlung auf Basis der defektbehafteten, d.h. nicht ursprünglichen, posterioren Kondylenlinie des Femurs kann zu nicht zufriedenstellenden Ergebnissen führen. Dem wirkt die Erfindung entgegen. Denn durch die erfindungsgemäße Lösung können Defekte an den posterioren Kondylen bei der Einstellung der I/E-Rotation und/oder der Ermittlung der erforderlichen Größe der Femurkomponente berücksichtigt werden. Es hat sich gezeigt, dass hierdurch verbesserte Ergebnisse erzielt werden können. Zu diesem Zweck weist das erfindungsgemäße chirurgische Instrument das wenigstens eine Kompensationselement auf. Das wenigstens eine Kompensationselement ist wahlweise an einem der beiden Referenzfüße, genauer: deren Fußoberseiten, anbringbar. Vereinfacht ausgedrückt, wird mittels des Kompensationselements gleichsam eine anteroposteriore Abmessung des Referenzfußes verändert und hierdurch der besagte Defekt maßlich ausgeglichen. In angebrachtem Zustand des Kompensationselements liegt anstelle der betreffenden Fußoberseite die Elementoberseite des Kompensationselements an der betreffenden (defektbehafteten) posterioren Kondyle an. Hierdurch werden der Defekt maßlich ausgeglichen und die ursprüngliche posteriore Kondylenlinie wiederhergestellt. Das wenigstens eine Kompensationselement bildet in angebrachtem Zustand eine form- und/oder kraftschlüssige Verbindung mit dem betreffenden Referenzfuß aus. Die Verbindung kann beispielsweise eine Steck-, Rast-, Klemm- und/oder Schnappverbindung sein. Die Verbindung ist lösbar und unmittelbar zwischen dem wenigstens einen Kompensationselement und dem betreffenden Referenzfuß ausgebildet, d.h. ohne die Zuhilfenahme eines gesonderten Verbindungsmittels. Der Referenzblock dient einer distalen Referenzierung an dem resezierten Femur. Hierfür weist der Referenzblock die proximal orientierte Blockrückseite auf. Diese liegt in der Verwendung des chirurgischen Instruments an der distalen Stirnfläche an. Die beiden Referenzfüße dienen einer Referenzierung an den posterioren Kondylen des Femurs. Zu diesem Zweck weisen die beiden Referenzfüße jeweils eine anterior orientierte Fußoberseite auf. Sofern das wenigstens eine Kompensationselement an keinem der Referenzfüße angebracht ist, liegen beide Fußoberseiten an jeweils einer der beiden posterioren Kondylen an. Bei einer Ausgestaltung sind die beiden Referenzfüße jeweils fest an dem Referenzblock angeordnet und/oder als Abschnitt desselben ausgebildet. Bei einer weiteren Ausgestaltung sind die Referenzfüße jeweils als separates Bauteil lösbar mit dem Referenzblock verbunden. Die Referenzfüße sind mediolateral voneinander beabstandet und können auch als lateraler Referenzfuß und medialer Referenzfuß bezeichnet werden. Dabei ist der laterale Referenzfuß zur Anlage an der lateralen posterioren Kondyle eingerichtet. Der mediale Referenzfuß ist dementsprechend zur Anlage an der medialen posterioren Kondyle eingerichtet. Der Taster dient der eigentlichen Ermittlung der Größe des Femurs und damit der erforderlichen Größe der zu implantierenden Femurkomponente. Zu diesem Zweck weist der Taster die Tasterspitze auf. Die Tasterspitze wird nach erfolgter distaler Referenzierung mittels der Blockrückseite und posteriorer Referenzierung mittels der Fußoberseiten (und gegebenenfalls des wenigstens einen Kompensationselements) zur Anlage an der anterioren Fläche des Femurs gebracht. Zu diesem Zweck ist die Tasterspitze vorzugsweise proximodistal und/oder anteroposterior relativbeweglich. Die erforderliche Größe kann beispielsweise an einer dem Taster zugeordneten Skale abgelesen werden. Bei einer Ausgestaltung ist der Taster fest mit dem Referenzblock verbunden. Bei einer weiteren Ausgestaltung ist der Taster abnehmbar an dem Referenzblock angebracht. Die Referenzbohrungen dienen der Aufnahme der besagten Referenzpins. Diese werden nach erfolgter Ausrichtung ausgehend von der Blockvorderseite durch die Referenzbohrungen in die distale Stirnfläche des resezierten Femurs eingebracht. Hiernach kann das chirurgische Instrument von den Referenzpins distal abgezogen werden. Die Referenzpins verbleiben femurseitig und dienen einer anschließenden Befestigung eines sogenannten Femurschnittblocks. Folglich gibt die Positionierung der Referenzbohrungen die spätere Ausrichtung des Femurschnittblocks und damit auch die Ausrichtung der mittels dessen an dem Femur anzubringenden Schnitte vor. Die Ausrichtung der Schnitte legt letztlich die I/E-Rotation der Femurkomponente fest. Bei einer Ausgestaltung sind die Referenzbohrungen unmittelbar in den Referenzblock eingebracht. Bei einer weiteren Ausgestaltung sind die Referenzbohrungen an einem separaten Bauteil ausgebildet, welches, vorzugsweise lösbar, mit dem Referenzblock verbindbar ist.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Rückseite" einer Komponente oder eines Abschnitts des chirurgischen Instruments, beispielsweise des Referenzblocks, in Bezug auf eine proximal gerichtete Blickrichtung verwendet. Dementgegen werden Bezeichnungen wie "Vorderseite" in Bezug auf eine distal gerichtete Blickrichtung verwendet.

Weiter gemäß der Erfindung weist das wenigstens eine Kompensationselement an seiner Elementunterseite eine anterior eingesenkte und posterior offene Aufnahmetasche auf, in welcher der Referenzfuß formschlüssig aufnehmbar ist. Die Aufnahmetasche ermöglicht eine besonders einfache und dennoch zuverlässige Anbringung des Kompensationselements an dem betreffenden Referenzfuß. Die Aufnahmetasche ist in die Elementunterseite eingesenkt, wobei die Elementunterseite der Elementoberseite posterior gegenüberliegt. Mit anderen Worten ist die Elementunterseite in angebrachtem Zustand des Kompensationselements der betreffenden Fußoberseite zugewandt. Die Aufnahmetasche ist posterior offen. Hierdurch kann das Kompensationselement in posteriorer Richtung auf den Referenzfuß aufgebracht werden. Die Aufnahmetasche bildet mit dem Referenzfuß eine Steck-, Rast- und/oder Klemmverbindung aus. Die Aufnahmetasche ist komplementär zu dem Referenzfuß und umgekehrt. Mit anderen Worten bildet die Aufnahmetasche eine Art Negativform des Referenzfußes. Bei einer alternativen Ausgestaltung weist das Kompensationselement einen anteroposterior zwischen der Elementunterseite und der Elementoberseite angeordneten Schlitz zum distalen Aufstecken auf den Referenzfuß auf.

In weiterer Ausgestaltung der Erfindung weist das wenigstens eine Kompensationselement an seiner Elementunterseite einen posterior aufragenden, insbesondere die Aufnahmetasche umgebenden, Außenrand auf, welcher formschlüssig mit einem Außenumfang des Referenzfußes verbindbar ist. In angebrachtem Zustand umgreift der Außenrand den Außenumfang des Referenzfußes. Hierdurch wird eine lösbare Steck-, Rast- und/oder Klemmverbindung zwischen dem wenigstens einen Kompensationselement und dem betreffenden Referenzfuß ausgebildet. Der Außenrand umgreift den Außenumfang des Referenzfußes wenigstens abschnittsweise. Sofern das wenigstens eine Kompensationselement eine Aufnahmetasche gemäß der vorhergehenden Ausgestaltung aufweist, bildet der Außenrand deren Berandung.

In weiterer Ausgestaltung der Erfindung ist der Außenrand elastisch nachgiebig. Die elastische Nachgiebigkeit kann werkstoff- und/oder gestaltbedingt sein. Beispielsweise kann der Außenrand, vorzugsweise das gesamte wenigstens eine Kompensationselement, aus einem elastisch nachgiebigen Werkstoff, beispielsweise einem Kunststoff, insbesondere einem Elastomer, gefertigt sein. Alternativ oder zusätzlich kann der Außenrand dünnwandig dimensioniert sein. Die dünnwandige Dimensionierung kann die besagte elastische Nachgiebigkeit bewirken oder wenigstens unterstützen. In angebrachtem Zustand des Kompensationselements bewirkt die elastische Nachgiebigkeit eine elastische Vorspannung auf den Außenumfang des Referenzfußes. Hierdurch wird einem ungewollten Lösen des Kompensationselements entgegengewirkt.

In weiterer Ausgestaltung der Erfindung weist das wenigstens eine Kompensationselement einen lateralen Elementabschnitt, einen medialen Elementabschnitt, einen Trennspalt, welcher die beiden Elementabschnitte voneinander trennt und proximodistal längserstreckt sowie einends offen ist, und einen Gelenkabschnitt auf, welcher andernends des Trennspalts angeordnet ist und die beiden Elementabschnitte elastisch gelenkbeweglich miteinander verbindet. Diese Ausgestaltung ermöglicht eine nochmals verbesserte Verbindung zwischen dem Kompensationselement und dem betreffenden Referenzfuß. Infolge des Trennspalts und des Gelenkabschnitts sind die beiden Elementabschnitte relativ zueinander elastisch beweglich. Eine solche elastische Beweglichkeit ist besonders vorteilhaft in Kombination mit der Aufnahmetasche und/oder dem Außenrand gemäß den vorhergehenden Ausgestaltungen. Bei einem Aufstecken, Aufrasten oder Aufklemmen des Kompensationselements auf den Referenzfuß federn die beiden Elementabschnitte quer zur Längserstreckung des Trennspalts auseinander. Die elastische Rückstellkraft verursacht zusätzliche Kontaktkräfte zwischen dem Kompensationselement und dem Referenzfuß. Die zusätzlichen Kontaktkräfte gehen mit einer erhöhten Reibung einher. Die erhöhte Reibung wirkt einem unbeabsichtigten Lösen des Kompensationselements von dem betreffenden Referenzfuß entgegen. Der laterale Elementabschnitt, der mediale Elementabschnitt und der Gelenkabschnitt sind einstückig. Der Gelenkabschnitt kann auch als Festkörpergelenk bezeichnet werden.

In weiterer Ausgestaltung der Erfindung sind mehrere unterschiedliche Kompensationselemente vorhanden, wobei die unterschiedlichen Kompensationselemente sich, vorzugsweise ausschließlich, im Hinblick auf eine jeweilige anteroposteriore Dicke unterscheiden. Mithilfe der unterschiedlichen Kompensationselemente können unterschiedlich stark ausgeprägte kondyläre Defekte ausgeglichen werden. Der unterschiedlich starken Ausprägung der Defekte wird durch die unterschiedlichen Dicken der Kompensationselemente Rechnung getragen. Ein in Relation schwach ausgeprägter Defekt kann mit einem vergleichsweise weniger dicken Kompensationselement ausgeglichen werden. Umgekehrt kann ein in Relation stark ausgeprägter Defekt mit einem vergleichsweise dicken Kompensationselement ausgeglichen werden. Abgesehen von ihren unterschiedlichen Dicken sind die unterschiedlichen Kompensationselemente vorzugsweise identisch. Bei einer Ausgestaltung sind die beiden Referenzfüße hinsichtlich ihrer Abmessungen und/oder Form identisch. In diesem Fall kann jedes der unterschiedlichen Kompensationselemente wahlweise an beiden Referenzfüßen angebracht werden. Bei einer weiteren Ausgestaltung weisen die beiden Referenzfüße eine unterschiedliche Form und/oder unterschiedliche Abmessungen auf. In diesem Fall kann den beiden Referenzfüßen jeweils ein gesonderter Satz unterschiedlicher Kompensationselemente zugeordnet sein.

In weiterer Ausgestaltung der Erfindung sind wenigstens drei unterschiedliche Kompensationselemente mit einer jeweiligen Dicke von 1 mm, 2 mm und 3 mm vorhanden. Die Erfinder haben erkannt, dass ein Wertebereich von 1 mm bis 3 mm und eine entsprechende Abstufung um jeweils 1 mm besonders vorteilhaft sind. Denn zum einen erlaubt der genannte Wertebereich die Kompensation deutlich unterschiedlich stark ausgeprägter Defekte. Zum anderen ermöglicht die genannte Abstufung es, die Anzahl der unterschiedlichen Kompensationselemente gering zu halten. Dies im Vergleich zu einer grundsätzlich denkbaren feineren Abstufung in Schritten von beispielsweise 0,5 mm.

In weiterer Ausgestaltung der Erfindung weist der Referenzblock eine zwischen der Blockrückseite und der Blockvorderseite durchgängige Apertur und ein in der Apertur angeordnetes sowie im Wesentlichen anteroposterior längserstrecktes Ausrichtelement auf, welches zum Ausrichten entlang der Whiteside-Linie des Femurs eingerichtet ist. Diese Ausgestaltung ermöglicht eine nochmals verbesserte Einstellung der I/E-Rotation. Die Apertur kann auch als Öffnung oder Sichtöffnung bezeichnet werden. In der Verwendung des chirurgischen Instruments erlaubt die Apertur dem ausführenden Operateur eine Sichtkontrolle des distalen Femurs. Mit anderen Worten kann der Operateur ausgehend von der Blockvorderseite durch die Apertur auf die an der Blockrückseite anliegende distale Stirnfläche des resezierten Femurs blicken. Das Ausrichtelement ist in der Apertur angeordnet und im Wesentlichen anteroposterior längserstreckt. Die I/E-Rotation wird eingestellt, indem der Referenzblock mitsamt des Ausrichtelements relativ zu der besagten Whiteside-Linie ausgerichtet wird. Die Whiteside-Linie bezeichnet die anteroposteriore Achse des distalen Femurs und kann auch als Trochlea-Achse bezeichnet werden. Die Whiteside-Linie ist orthogonal zu der sogenannten transepikondylären Achse des distalen Femurs ausgerichtet. Hiervon zu unterscheiden ist die posteriore Kondylenlinie. In Bezug auf die gemeinsame Referenzebene der Fußoberseiten - und damit auch in Bezug auf die virtuelle Abstandslinie zwischen den Referenzbohrungen - ist das Ausrichtelement bei unterschiedlichen Ausgestaltungen unterschiedlich orientiert. Bei einer Ausgestaltung liegt eine orthogonale Orientierung vor. Dies entspricht einer (neutralen) I/E-Rotation von 0°. Bei einer Ausgestaltung liegt eine Abweichung von der Orthogonalen um 3°, bei einer weiteren Ausgestaltung um 5° vor. Dies entspricht einer I/E-Rotation von 3° bzw. 5°. Je nach Vorzeichen kann eine interne oder eine externe Rotation eingestellt werden. Bei einer weiteren Ausgestaltung ist die Orientierung des Ausrichtelements in Bezug auf die gemeinsame Referenzebene und/oder virtuelle Abstandslinie, vorzugsweise abgestuft, veränderlich.

In weiterer Ausgestaltung der Erfindung ist das Ausrichtelement relativ zu dem Referenzblock beweglich zwischen einer ersten Stellung, in welcher das Ausrichtelement in einem ersten Winkel zu der virtuellen Abstandslinie der Referenzbohrungen orientiert ist, und einer zweiten Stellung, in welcher das Ausrichtelement in einem zweiten Winkel zu der virtuellen Abstandslinie der Referenzbohrungen orientiert ist. Durch eine Verlagerung des Ausrichtelements zwischen der ersten Stellung und der zweiten Stellung kann die I/E-Rotation wahlweise entsprechend eingestellt werden. Alternativ ist es denkbar, dass der erste Winkel und der zweite Winkel betragsmäßig identisch und mit unterschiedlichen Vorzeichen behaftet sind. Hierdurch kann das chirurgische Instrument in der ersten Stellung des Ausrichtelements für das linke Knie und in der zweiten Stellung für das rechte Knie verwendet und jeweils eine betragsmäßig identische I/E-Rotation eingestellt werden.

In weiterer Ausgestaltung der Erfindung weist das Ausrichtelement ein posteriores Ende, welches an dem Referenzblock um eine proximodistal orientierte Schwenkachse beweglich gelagert ist, und ein anteriores Ende auf, welches über die Blockoberseite ragt und zur manuellen Bewegung des Ausrichtelements zwischen der ersten Stellung und der zweiten Stellung eingerichtet ist. Diese Ausgestaltung ermöglicht eine besonders zuverlässige und ergonomische Einstellbarkeit des Ausrichtelements und damit der I/E-Rotation. Zu diesem Zweck ist das Ausrichtelement einends, an seinem posterioren Ende, an dem Referenzblock gelagert. Genauer ist das Ausrichtelement um die proximodistal orientierte Schwenkachse schwenkbeweglich an dem Referenzblock gelagert. Die Schwenkachse ist bei einer Ausgestaltung eine gedachte geometrische Achse. Bei einer weiteren Ausgestaltung ist die Schwenkachse ein physisch vorhandenes Bauteil. Die Schwenkachse ist orthogonal zu der Blockrückseite orientiert. Das der Schwenkachse abgewandte, anteriore Ende des Ausrichtelements dient der manuellen Bewegung. Zur besseren manuellen Erreichbarkeit ragt das anteriore Ende über die Blockoberseite hinaus.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivdarstellung eine Ausführungsform eines erfindungsgemäßen chirurgischen Instruments,
- Fig. 2: eine weitere schematische Perspektivdarstellung des chirurgischen Instruments nach Fig. 1,
- Fig. 3: eine vergrößerte Detailansicht des chirurgischen Instruments in einem Detailbereich A nach Fig. 2,
- Fig. 4: in schematischer Perspektivdarstellung eine exemplarische intraoperative Situation, in welcher das chirurgische Instrument an einen resezierten distalen Femur angelegt ist,
- Fig. 5: der resezierte Femur nach Fig. 4 in einer proximal gerichteten Blickrichtung und unter Ausblendung des chirurgischen Instruments,
- Fig. 6, 7, 8: vergrößerte Detaildarstellungen eines Kompensationselements des chirurgischen Instruments in einer schematischen Perspektivdarstellung (Fig. 6), einer schematischen Aufsicht (Fig. 7) und einer schematischen Untersicht (Fig. 8),
- Fig. 9, 10: unterschiedliche schematische Perspektivdarstellungen einer Variante des Kompensationselements nach den Fig. 6 bis 8,
- Fig. 11: eine ausschnittsweise Darstellung des chirurgischen Instruments in einer Konfiguration, in welcher zwei unterschiedliche Kompensationselemente vorhanden sind, und
- Fig. 12: die Konfiguration nach Fig. 11 in einer weiteren schematischen Perspektivdarstellung.

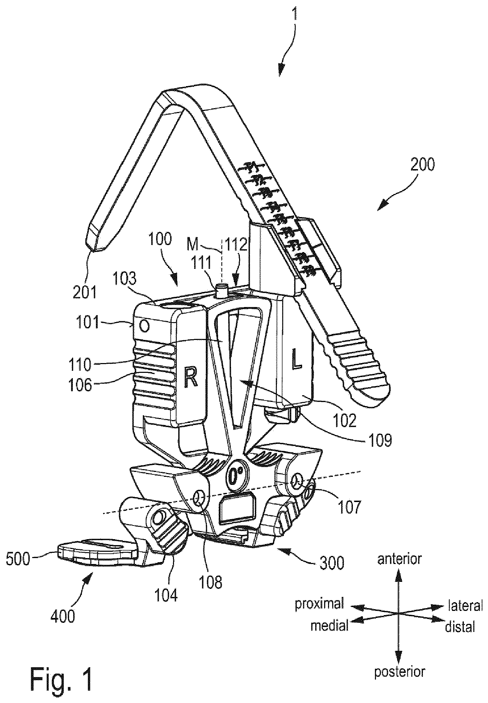

Gemäß den Fig. 1 bis 4 ist ein chirurgisches Instrument 1 zur Verwendung bei einer Kniegelenkersatzoperation vorgesehen und weist einen Referenzblock 100, einen Taster 200, zwei Referenzfüße 300, 400, zwei Referenzbohrungen 107, 108 sowie wenigstens ein Kompensationselement 500 auf.

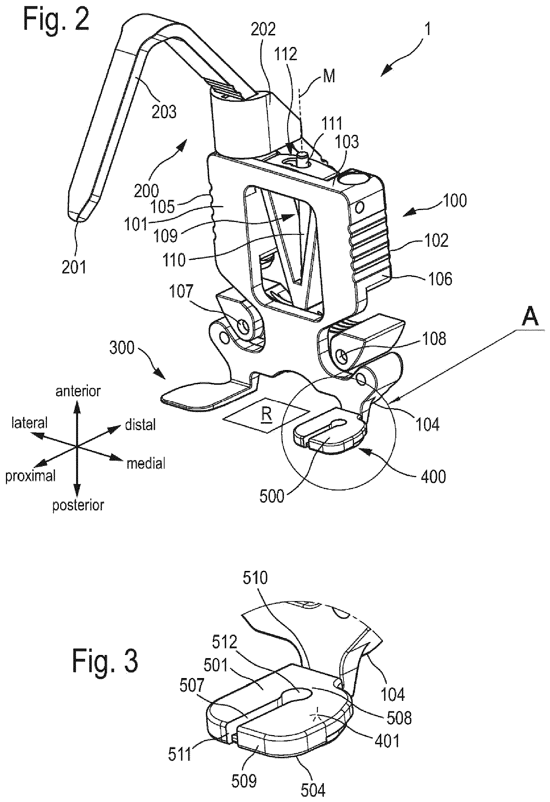

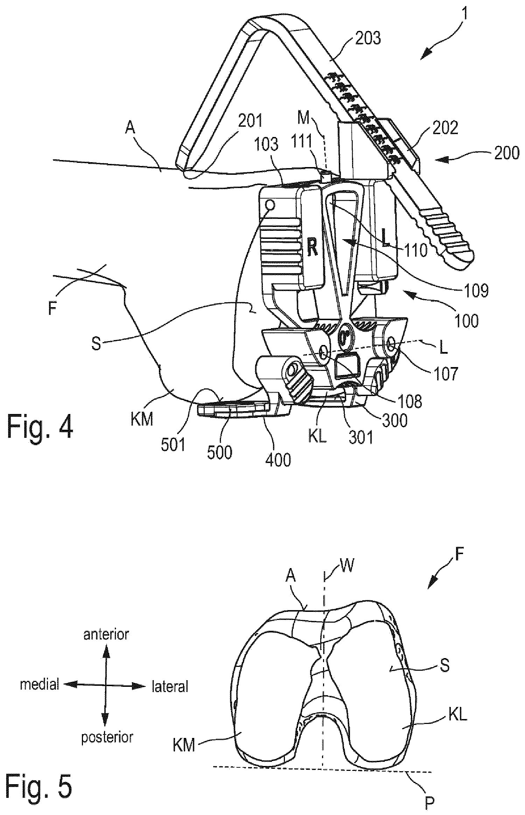

Der Referenzblock 100 weist eine proximal orientierte Blockrückseite 101, eine distal gegenüberliegende Blockvorderseite 102, eine anteriore Blockoberseite 103, eine posteriore Blockunterseite 104, eine laterale Blockaußenseite 105, und eine mediale Blockaußenseite 106 auf. Die Blockrückseite 101 ist zur Anlage an einer distalen Stirnfläche S eines resezierten Femurs F eingerichtet (siehe Fig. 4). Eine Normalenrichtung der Blockrückseite 101 ist parallel zu der in den Fig. 1 und 2 eingezeichneten proximodistalen Achse orientiert und weist in proximale Richtung.

Der Taster 200 ist im Bereich der anterioren Blockoberseite 103 an dem Referenzblock 100 angebracht und weist eine Tasterspitze 201 auf. Die Tasterspitze 201 ragt proximal über die Blockrückseite 101 und ist zur Anlage an einer anterioren Fläche A des Femurs eingerichtet (siehe Fig. 4). Bei der vorliegenden Ausführungsform ist der Taster 200 auf noch näher beschriebene Weise lösbar mit dem Referenzblock 100 verbunden. Bei einer zeichnerisch nicht dargestellten Ausführungsform ist der Taster fest mit dem Referenzblock verbunden.

Die beiden Referenzfüße 300, 400 werden nachfolgend auch als lateraler Referenzfuß 300 und medialer Referenzfuß 400 bezeichnet. Die beiden Referenzfüße 300, 400 sind entlang der in den Fig. 1 und 2 eingezeichneten mediolateralen Achse voneinander beabstandet und jeweils im Bereich der Blockunterseite 104 des Referenzblocks 100 angeordnet. Bei der gezeigten Ausführungsform sind die beiden Referenzfüße 300, 400 jeweils fest an dem Referenzblock 100 angeordnet. Im Speziellen bilden die Referenzfüße 300, 400 jeweils einen Abschnitt des Referenzblocks 100. Bei einer in den Figuren nicht dargestellten Ausführungsform sind die Referenzfüße jeweils ein gesondertes Bauteil, das lösbar mit dem Referenzblock verbunden ist. Die beiden Referenzfüße 300, 400 weisen jeweils eine anterior orientierte und proximal über die Blockrückseite 101 ragende Fußoberseite 301, 401 auf. Die Fußoberseiten 301, 401 werden nachfolgend auch als laterale Fußoberseite 301 (des lateralen Referenzfußes 300) und mediale Fußoberseite 401 (des medialen Referenzfußes 400) bezeichnet. Die beiden Fußoberseiten 301, 401 sind in Bezug auf die anteroposteriore Achse auf gleicher Höhe und insoweit in einer gemeinsamen Ebene angeordnet. Die besagte Ebene wird als Referenzebene R bezeichnet (siehe Fig. 2). Die Fußoberseiten 301, 401 sind orthogonal zu der Blockrückseite 101 orientiert und jeweils zur Anlage an einer posterioren Kondyle KM, KL (siehe Fig. 4, 5) eingerichtet. Die laterale Fußoberseite 301 ist zur Anlage an der lateralen posterioren Kondyle KL eingerichtet. Die mediale Fußoberseite 401 ist zur Anlage an der medialen posterioren Kondyle KM eingerichtet (siehe Fig. 5).

Die beiden Referenzbohrungen 107, 108 sind jeweils orthogonal zu der Blockrückseite 101 durchgängig zwischen der Blockrückseite 101 und der Blockvorderseite 102 erstreckt. Die Referenzbohrungen 107, 108 sind mediolateral voneinander beabstandet und werden nachfolgend auch als laterale Referenzbohrung 107 und mediale Referenzbohrung 108 bezeichnet. Die Referenzbohrungen 107, 108 sind entlang einer virtuellen Abstandslinie L voneinander beabstandet. Die virtuelle, d.h. gedachte, Abstandslinie L ist zwischen nicht näher bezeichneten Bohrungsmittelpunkten der Referenzbohrungen 107, 108 längserstreckt. Die virtuelle Abstandslinie L ist parallel zu den Fußoberseiten 301, 401 und damit auch zu der Referenzebene R längserstreckt. Die Referenzbohrungen 107, 108 sind jeweils zur Aufnahme eines in den Figuren nicht näher gezeigten Referenzpins eingerichtet. Die besagten Referenzpins sind beispielsweise in der anhand Fig. 4 gezeigten intraoperativen Situation ausgehend von der Blockvorderseite 102 in proximaler Richtung durch die jeweilige Referenzbohrung 107, 108 in die distale Stirnfläche S einbringbar. Nach Entfernen des chirurgischen Instruments 1 von dem Femur F verbleiben die Referenzpins an Ort und Stelle und dienen der Befestigung eines Femurschnittblocks (englisch: femoral cutting jig). Hierauf wird nachfolgend noch näher eingegangen. Bei der gezeigten Ausführungsform sind die Referenzbohrungen 107, 108 unmittelbar durch den Referenzblock 100 erstreckt und/oder in denselben eingebracht. Bei einer in den Figuren nicht gezeigten Ausführungsform sind die Referenzbohrungen in ein gesondertes Bauteil eingebracht, welches, vorzugsweise lösbar, mit dem Referenzblock verbunden ist.

Das wenigstens eine Kompensationselement 500 ist in der in den Fig. 1 bis 4 gezeigten Konfiguration an dem medialen Referenzfuß 400 angebracht. Das Kompensationselement 500 ist zu diesem Zweck zur kraft- und/oder formschlüssigen lösbaren Verbindung mit dem medialen Referenzfuß 400 eingerichtet. Die besagte lösbare Verbindung ist unmittelbar zwischen dem Kompensationselement 500 und dem medialen Referenzfuß 400 ausgebildet, d.h. ohne zusätzliche Verbindungsmittel hergestellt. Dies auf noch näher beschriebene Weise. Das Kompensationselement 500 weist eine anterior orientierte Elementoberseite 501 auf (siehe insbesondere Fig. 3). Die Elementoberseite 501 ist planparallel zu der Referenzebene R und damit orthogonal zu der Blockrückseite 101 orientiert. In der Verwendung des chirurgischen Instruments 1 gelangt - je nachdem, ob das Kompensationselement 500 an dem medialen Referenzfuß 400 angebracht ist oder nicht - entweder die mediale Fußoberseite 401 oder an deren Stelle die Elementoberseite 501 zur Anlage an der medialen Kondyle KM.

Die Funktion des chirurgischen Instruments 1 und insbesondere der Zweck des wenigstens einen Kompensationselements 500 werden nachfolgend im Detail exemplarisch anhand der intraoperativen Situation gemäß Fig. 4 erläutert.

Das chirurgische Instrument 1 dient mehreren Zwecken. Zum einen kann mittels des chirurgischen Instruments 1 eine Größenmessung und/oder Kontrolle an dem distal resezierten Femur F durchgeführt werden. Zum anderen dient das chirurgische Instrument 1 einer Einstellung der sogenannten I/E-Rotation. Im Einzelnen:
Das chirurgische Instrument 1 wird nach Anbringung eines distalen Femurschnitts an die resultierende Stirnfläche S angelegt (siehe Fig. 4). Hierbei kontaktiert die Blockrückseite 101 die Stirnfläche S. Die beiden Referenzfüße 300, 400 untergreifen die posterioren Kondylen KM, KL.

Zur weiteren Erläuterung werden nachfolgend zwei exemplarische Zustände unterschieden.

In einem ersten Zustand weisen die posterioren Kondylen KM, KL keinen Defekt, d.h. insbesondere keine Knorpel- und/oder Knochenabnutzung, auf. In dem ersten Zustand verläuft die sogenannte posteriore Kondylenlinie P wie in Fig. 5 dargestellt. Dieser Zustand der posterioren Kondylenlinie P kann auch als "ursprünglich", d.h. nicht durch etwaige kondyläre Defekte beeinflusst, bezeichnet werden.

In einem zweiten Zustand weist die mediale Kondyle KM einen in den Figuren nicht im Detail gezeigten Defekt auf. In dem zweiten Zustand weicht die posteriore Kondylenlinie im Bereich der Kondyle KM ausgehend von der in Fig. 5 gezeigten ursprünglichen Situation in Bezug auf die Zeichenebene nach oben ab. Mit anderen Worten ausgedrückt ist die posteriore Kondylenlinie in dem zweiten Zustand um eine proximodistale Achse rotiert.

In dem ersten Zustand kann eine posteriore Referenzierung ohne das Kompensationselement erfolgen. Hierbei wird die laterale Fußoberseite 301 an die laterale posteriore Kondyle KL angelegt und die mediale Fußoberseite 401 wird an die mediale posteriore Kondyle KM angelegt. Die (ursprüngliche) posteriore Kondylenlinie P erstreckt sich in diesem Fall in der Referenzebene R. Die virtuelle Abstandslinie L ist zu dieser parallel längserstreckt. Nach Setzen der besagten Referenzpins durch die Referenzbohrungen 107, 108, Entfernen des chirurgischen Instruments 1 und Anbringung des Femurschnittblocks ist dieser in einer (neutralen) I/E-Rotation mit 0° in Bezug auf die proximodistale Achse ausgerichtet. Der Einfachheit halber wird nachfolgend davon ausgegangen, dass eine solche Ausrichtung insbesondere im Rahmen einer KA wünschenswert ist und mit besonderen chirurgischen/medizinischen Vorteilen einhergeht.

In dem defektbehafteten zweiten Zustand kann eine solche (neutrale) I/E-Rotation nicht ohne weiteres gewährleistet werden. Denn durch die vorbeschriebene Abweichung der posterioren Kondylenlinie von ihrem ursprünglichen Zustand ergibt sich naturgemäß eine Rotation der virtuellen Abstandslinie L und folglich auch eine Rotation des Femurschnittblocks. Um dem entgegenzuwirken, ist das wenigstens eine Kompensationselement 500 vorhanden. Zur Kompensation des besagten Defekts wird das Kompensationselement 500 vorliegend an der medialen posterioren Kondyle KM angebracht. Anstelle der medialen Fußoberseite 401 gelangt nun die Elementoberseite 501 zur Anlage an der medialen posterioren Kondyle KM. Hierdurch wird der Defekt maßlich ausgeglichen und die posteriore Referenzierung erfolgt im Ergebnis so, als liege nach wie vor die ursprüngliche posteriore Kondylenlinie P vor.

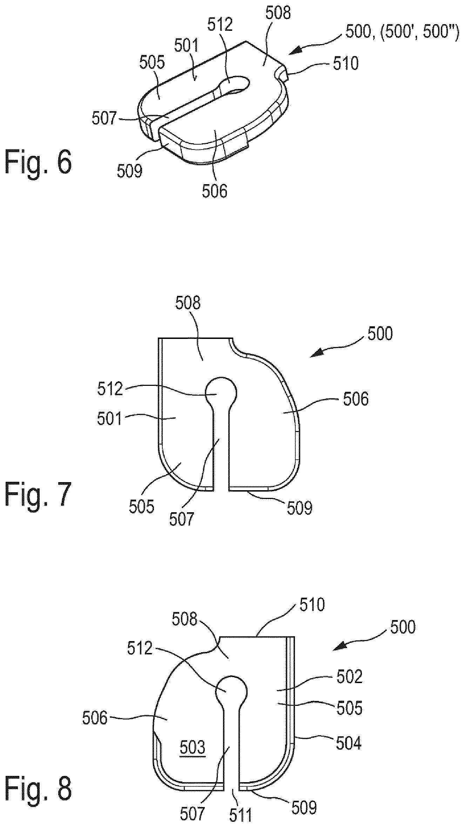

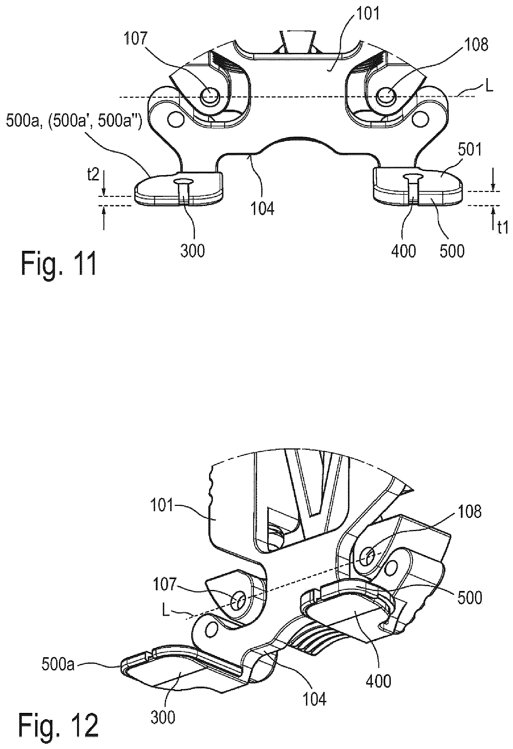

Zur maßlichen Kompensation unterschiedlich stark ausgeprägter Defekte weist das chirurgische Instrument vorliegend nicht lediglich das in den Fig. 1 bis 4 gezeigte Kompensationselement 500 auf. Stattdessen sind mehrere unterschiedliche Kompensationselemente 500, 500', 500" mit unterschiedlicher anteroposteriorer Dicke vorhanden. Die weiteren Kompensationselemente 500', 500" sind in den Figuren nicht gesondert dargestellt und weisen abgesehen von der unterschiedlichen anteroposterioren Dicke eine zu dem Kompensationselement 500 identische Gestalt und Funktionsweise auf. Zudem versteht es sich, dass eine Kompensation nicht lediglich im Bereich der medialen posterioren Kondyle KM, sondern alternativ oder zusätzlich an der lateralen posterioren Kondyle KL erfolgen kann. Zu diesem Zweck weist das chirurgische Instrument 1 vorliegend weitere Kompensationselemente 500a, 500a', 500a" auf, die an dem lateralen Referenzfuß 300 anbringbar sind. In den Fig. 11 und 12 ist ein solches Kompensationselement 500a gezeigt. Das Vorhandensein der weiteren Kompensationselemente 500', 500" und 500a', 500a" ist durch die in den Fig. 6 sowie 11 in Klammern gesetzten Bezugszeichen verdeutlicht. Im Übrigen ist in den Fig. 11 und 12 eine exemplarische Situation gezeigt, in welcher unterschiedlich dicke Kompensationselemente verwendet werden. Dabei weist das Kompensationselement 500 eine erste Dicke t1 auf. Das lateral angebrachte Kompensationselement 500a weist eine zweite Dicke t2 auf.

Zur Größenmessung und/oder -kontrolle des Femurs F wird die Tasterspitze 201 zur Anlage an der anterioren Fläche A gebracht. Vorliegend ist die Tasterspitze 201 zu diesem Zweck relativbeweglich gelagert. Im Speziellen weist der Taster 200 vorliegend eine Tasterbasis 202 und einen Tasterstab 203 auf, an welchem einends die Tasterspitze 201 angeordnet ist. Der Tasterstab 203 ist entlang einer nicht näher bezeichneten Führungsachse gleitbeweglich an der Tasterbasis 202 geführt. Die Tasterbasis 202 ist lösbar an dem Referenzblock 100 angebracht. Der weitere Aufbau und die Funktion des Tasters im Speziellen stehen nicht im Mittelpunkt der vorliegenden Erfindung. Deshalb werden weitere diesbezügliche Ausführungen als entbehrlich erachtet.

Nachfolgend werden weitere Merkmale des Kompensationselements 500 und dessen Funktion im Detail erläutert. Das zu dem Kompensationselement 500 Gesagte gilt mutatis mutandis auch für die weiteren Kompensationselemente 500', 500" sowie 500a, 500a' und 500a".

Zur lösbaren Anbringung des Kompensationselements 500 sind grundsätzlich unterschiedliche Arten von Fügeverbindungen denkbar. Beispielsweise zu nennen sind Steck-, Rast-, Klemm- und/oder Schnappverbindungen.

Bei der gezeigten Ausführungsform ist das Kompensationselement 500 auf den Referenzfuß 400 kraft- und/oder formschlüssig aufsteckbar. Zu diesem Zweck weist das Kompensationselement 500 an einer der Elementoberseite 501 posterior gegenüberliegenden Elementunterseite 502 eine Aufnahmetasche 503 auf (siehe Fig. 8). Die Aufnahmetasche 503 ist maßlich auf die Abmessungen des medialen Referenzfußes 400 abgestimmt. Der mediale Referenzfuß 400 ist kraft- und/oder formschlüssig in der Aufnahmetasche 503 aufnehmbar.

In angebrachtem Zustand überdeckt die Elementoberseite 501 die mediale Fußoberseite 401. Aufgrund der vorliegenden Dicke t1 ist die Elementoberseite 501 dementsprechend anterior beabstandet - in Bezug auf die Zeichenebene der Fig. 1 - oberhalb der medialen Fußoberseite 401 angeordnet.

Das Kompensationselement 500 weist bei der gezeigten Ausführungsform zudem einen Außenrand 504 auf. Der Außenrand 504 ragt in Normalenrichtung der Elementunterseite 502 von derselben auf. In Bezug auf die in den Fig. 1 bis 4 gezeigte Konfiguration ragt der Außenrand 504 posterior von der Elementunterseite 502 auf. Der Außenrand 504 ist formschlüssig mit einem Außenumfang (ohne Bezugszeichen) des medialen Referenzfußes 400 verbindbar. Zu diesem Zweck ist der Außenrand 504 komplementär zu dem besagten Außenumfang geformt und umgekehrt. Der Außenrand 504 umgreift den Außenumfang des Referenzfußes 400, so dass das Kompensationselement 500 nach Art eines Deckels auf den Referenzfuß 400 aufgebracht ist.

Bei der gezeigten Ausführungsform bildet der Außenrand 504 gleichsam eine äußere Begrenzung der Aufnahmetasche 503.

Die Aufnahmetasche 503 ist bei der gezeigten Ausführungsform zum einen posterior offen. Zum anderen ist die Aufnahmetasche 503 - jedenfalls in Bezug auf die in den Fig. 1 bis 4 gezeigte Konfiguration - in distaler Richtung offen. Hierdurch kann das Kompensationselement 500 distal auf den Referenzfuß 400 aufgeschoben werden. Alternativ kann das Kompensationselement 500 posterior aufgesteckt werden. Der Außenrand 504 begrenzt die Aufnahmetasche 503 vorliegend mediolateral sowie proximal.

Das in den Fig. 5 bis 8 im Detail gezeigte Kompensationselement 500 weist zudem einen lateralen Elementabschnitt 505, einen medialen Elementabschnitt 506, einen Trennspalt 507 und einen Gelenkabschnitt 508 auf.

Der Trennspalt 507 separiert die beiden Elementabschnitte 505, 506 voneinander und ist vorliegend parallel zu der in den Fig. 1 und 2 eingezeichneten proximodistalen Achse längserstreckt. Dabei ist der Trennspalt 507 einends offen. Vorliegend ist der Trennspalt ausgehend von einer Elementrückseite 509 in das Kompensationselement 500 hineinerstreckt. Ausgehend von seinem offenen Ende ist der Trennspalt 507 in Richtung einer Elementvorderseite 510 längserstreckt. Die Elementvorderseite 510 liegt der Elementrückseite 509 distal gegenüber. Vorliegend weist der Trennspalt 507 seiner Öffnung 511 abgewandt eine Aufweitung 512 auf.

Der Gelenkabschnitt 508 ist an dem der Öffnung 511 abgewandten Ende des Trennspalts 507 angeordnet und verbindet die beiden Elementabschnitte 505, 506 elastisch gelenkbeweglich miteinander. Der Gelenkabschnitt 508 ermöglicht ein begrenztes elastisches Auffedern des Kompensationselements 500 in Bezug auf die mediolaterale Achse (siehe insbesondere Fig. 3, 4). Hierdurch werden die beiden Elementabschnitte 505, 506 beim Aufstecken des Kompensationselements 500 auf den medialen Referenzfuß 400 elastisch gegeneinander aufgespreizt. Hiernach liegt der Außenrand 504 elastisch vorgespannt an dem Außenumfang des Referenzfußes 400 an. Die elastische Vorspannung wirkt einem ungewollten Ablösen des Kompensationselements 500 entgegen. Der Gelenkabschnitt 508 bildet vorliegend eine Art Festkörpergelenk mit einer anteroposterior orientierten Gelenkachse.

Bei der gezeigten Ausführungsform ist das Kompensationselement 500 aus einem für eine medizinische Verwendung geeigneten Kunststoff hergestellt. Solche Kunststoffe sind dem Fachmann bekannt. Alternativ kann das Kompensationselement aus Metall gefertigt sein.

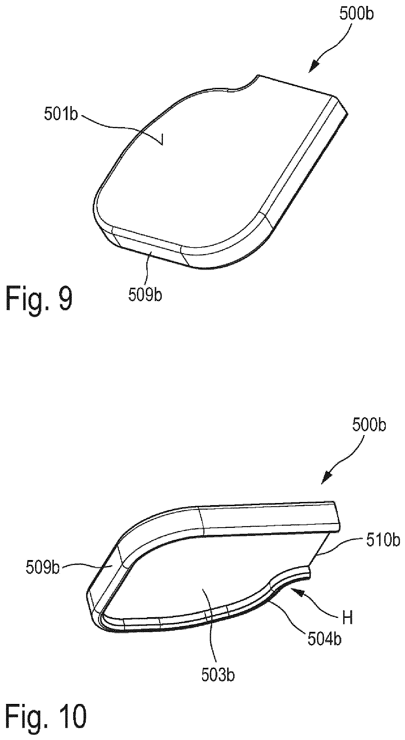

Die Fig. 9 und 10 zeigen ein Kompensationselement 500b. Das Kompensationselement 500b ist eine Variante des Kompensationselements 500 nach den Fig. 6 bis 8. Zur Vermeidung von Wiederholungen werden nachfolgend lediglich wesentliche Unterschiede des Kompensationselements 500b im Vergleich zu dem Kompensationselement 500 erläutert. Identische Merkmale werden nicht nochmals erörtert. Stattdessen wird auf die Beschreibung im Zusammenhang mit den Fig. 6 bis 8 ausdrücklich Bezug genommen und verwiesen.

Das Kompensationselement 500b weist im Unterschied zu dem Kompensationselement 500 keinen Trennspalt auf. Zudem erlaubt das Kompensationselement 500b kein Aufstecken in distaler Richtung. Stattdessen kann das Kompensationselement 500b lediglich posterior aufgesteckt werden. Insoweit ist wiederum eine Aufnahmetasche 503b vorhanden. Diese wird von einem Außenrand 504b umrandet. Der Außenrand 504b ist vorliegend elastisch nachgiebig. Die elastische Nachgiebigkeit des Außenrands 504b kann werkstoff- und/oder gestaltbedingt sein. Vorliegend tragen zum einen die Werkstoffwahl und zum anderen die vergleichsweise dünnwandige Gestaltung des Außenrands 504b zu dessen elastischer Nachgiebigkeit bei. Durch die elastische Nachgiebigkeit des Außenrands 504b wird eine verbesserte Klemmung auf dem betreffenden Referenzfuß erreicht. Der Außenrand 504b weist vorliegend einen Hinterschnitt H auf (siehe Fig. 10). Der Hinterschnitt H verhindert, dass das Kompensationselement 500b distal auf den betreffenden Referenzfuß aufgesteckt werden kann. Gleichzeitig ist einem proximalen Abziehen des Kompensationselements 500b entgegengewirkt.

Weiter in Bezug auf die Fig. 1 bis 5 weist das chirurgische Instrument 1 vorliegend im Bereich seines Referenzblocks 100 eine Apertur 109 und ein in der Apertur 109 angeordnetes Ausrichtelement 110 auf.

Die Apertur 109 ist proximodistal durchgängig zwischen der Blockrückseite 101 und der Blockvorderseite 102 erstreckt und bildet eine Sichtöffnung, durch welche der Operateur in proximaler Blickrichtung auf die distale Stirnfläche S blicken kann. Die Apertur 109 ist vorliegend in etwa dreieckig geformt. Alternative Formen sind selbstverständlich denkbar.

Das Ausrichtelement 110 ist anteroposterior längserstreckt und in Bezug auf die mediolaterale Achse in etwa mittig in der Apertur 109 angeordnet. Das Ausrichtelement 110 dient einem Ausrichten des chirurgischen Instruments 1 entlang der sogenannten Whiteside-Linie W (siehe Fig. 5). Die Whiteside-Linie W bildet ebenso wie die posteriore Kondylenlinie P eine anatomische Landmarke zur Ausrichtung des chirurgischen Instruments 1 an dem Femur F. Mithilfe des Ausrichtelements 110 kann der Operateur das chirurgische Instrument 1 in der anhand Fig. 4 gezeigten intraoperativen Situation in Bezug auf die Whiteside-Linie W ausrichten. Hierzu wird das Ausrichtelement 110 - in Bezug auf eine posterior gerichtete Blickrichtung - in Überdeckung mit der Whiteside-Linie W gebracht.

Das Ausrichtelement 110 ist entlang seiner Längsachse M zwischen einem in den Figuren nicht näher gezeigten posterioren Ende und einem anterioren Ende 111 längserstreckt. Die beiden Enden können auch als erstes Ende und zweites Ende 111 bezeichnet werden. Das zweite Ende 111 ragt anterior über die Blockoberseite 103 hinaus. Das erste Ende ist auf nicht näher gezeigte Weise um eine proximodistal orientierte Schwenkachse (ohne Bezugszeichen) beweglich an dem Referenzblock 100 gelagert. Hierdurch kann das Ausrichtelement 110 über eine Manipulation an dem zweiten Ende 111 zwischen unterschiedlichen Stellungen bewegt werden.

Vorliegend ist eine Bewegung zwischen einer in den Figuren gezeigten ersten Stellung und einer nicht näher gezeigten zweiten Stellung möglich. In der ersten Stellung ist die Längsachse M um 3° gegenüber der Normalenrichtung der Referenzebene R geneigt (siehe Fig. 2). In der zweiten Stellung beträgt die Neigung 5°. Das zweite Ende 111 ist zwischen den beiden Stellungen beweglich in einer Aufnahmeaussparung 112 des Referenzblocks 100 gehalten (siehe Fig. 2). Die Aufnahmeaussparung ist vorliegend als Langloch geformt. Das zweite Ende 111 ist in den beiden Stellungen des Ausrichtelements 110 entweder an dem einen oder dem anderen Ende des Langlochs bzw. der Aufnahmeaussparung 112 gehalten.

Die Beweglichkeit des Ausrichtelements 110 ermöglicht eine angepasste Einstellung der I/E-Rotation. Vorliegend kann in der ersten Stellung des Ausrichtelements 110 eine I/E-Rotation von 3° eingestellt werden. In der zweiten Stellung ergibt sich eine I/E-Rotation von 5°.

## Patentansprüche

1. Chirurgisches Instrument (1) zur Verwendung bei einer Kniegelenkersatzoperation, aufweisend
einen Referenzblock (100) mit einer proximal orientierten Blockrückseite (101), welche zur Anlage an einer distalen Stirnfläche (S) eines resezierten Femurs (F) eingerichtet ist,
einen Taster (200), welcher im Bereich einer anterioren Blockoberseite (103) des Referenzblocks (100) an demselben angeordnet ist und eine Tasterspitze (201) aufweist, welche proximal über die Blockrückseite (101) ragt und zur Anlage an einer anterioren Fläche (A) des Femurs eingerichtet ist,
zwei mediolateral voneinander beabstandete Referenzfüße (300, 400), welche jeweils im Bereich einer der Blockoberseite (103) posterior gegenüberliegenden Blockunterseite (104) des Referenzblocks (100) an demselben angeordnet sind und jeweils eine anterior orientierte sowie proximal über die Blockrückseite (101) ragende Fußoberseite (301, 401) aufweisen, wobei die Fußoberseiten (301, 401) in einer gemeinsamen Referenzebene (R) angeordnet sind und jeweils zur Anlage an einer posterioren Kondyle (KL, KM) des Femurs (F) eingerichtet sind,
wenigstens zwei Referenzbohrungen (107, 108), welche jeweils orthogonal zu der Blockrückseite (101) durchgängig von derselben bis auf eine distal gegenüberliegende Blockvorderseite (103) erstreckt und jeweils zur Aufnahme eines in die distale Stirnfläche (S) des Femurs (F) einbringbaren Referenzpins eingerichtet sind, wobei die wenigstens zwei Referenzbohrungen (107, 108) entlang einer virtuellen Abstandslinie (L), welche mediolateral und parallel zu der Referenzebene (R) längserstreckt ist, voneinander beabstandet sind,
wobei wenigstens ein Kompensationselement (500) vorhanden und zur form- und/oder kraftschlüssigen lösbaren Verbindung mit einem der beiden Referenzfüße (300, 400) eingerichtet ist, und wobei das wenigstens eine Kompensationselement (500) eine anterior orientierte Elementoberseite (501) aufweist, welche anstelle der betreffenden Fußoberseite (301, 401) zur Anlage an der betreffenden posterioren Kondyle (KL, KM) eingerichtet ist,
**dadurch gekennzeichnet, dass** das wenigstens eine Kompensationselement (500) an seiner Elementunterseite (502) eine anterior eingesenkte und posterior offene Aufnahmetasche (503) aufweist, in welcher der Referenzfuß (300, 400) formschlüssig aufnehmbar ist.

2. Chirurgisches Instrument (1) nach Anspruch 1, **dadurch gekennzeichnet, dass** das wenigstens eine Kompensationselement (500) an seiner Elementunterseite (502) einen posterior aufragenden, insbesondere die Aufnahmetasche (503) umgebenden, Außenrand (504) aufweist, welcher formschlüssig mit einem Außenumfang des Referenzfußes (300, 400) verbindbar ist.

3. Chirurgisches Instrument (1) nach Anspruch 2, **dadurch gekennzeichnet, dass** Außenrand (504) elastisch nachgiebig ist.

4. Chirurgisches Instrument (1) nach einem der vorhergehenden Ansprüche, **dadurch gekennzeichnet, dass** das wenigstens eine Kompensationselement (500) einen lateralen Elementabschnitt (505), einen medialen Elementabschnitt (506), einen Trennspalt (507), welcher die beiden Elementabschnitte (505, 506) voneinander trennt und proximodistal längserstreckt sowie einends offen ist, und einen Gelenkabschnitt (508) aufweist, welcher andernends des Trennspalts (507) angeordnet ist und die beiden Elementabschnitte (505, 506) elastisch gelenkbeweglich miteinander verbindet.

5. Chirurgisches Instrument (1) nach einem der vorhergehenden Ansprüche, **dadurch gekennzeichnet, dass** mehrere unterschiedliche Kompensationselemente (500, 500', 500") vorhanden sind, wobei die unterschiedlichen Kompensationselemente (500, 500', 500") sich, vorzugsweise ausschließlich, im Hinblick auf eine jeweilige anteroposteriore Dicke (t1, t2) unterscheiden.

6. Chirurgisches Instrument (1) nach Anspruch 5, **dadurch gekennzeichnet, dass** wenigstens drei unterschiedliche Kompensationselemente (500, 500', 500") mit einer jeweiligen Dicke von 1 mm, 2 mm und 3 mm vorhanden sind.

7. Chirurgisches Instrument (1) nach einem der vorhergehenden Ansprüche, **dadurch gekennzeichnet, dass** der Referenzblock (100) eine zwischen der Blockrückseite (101) und der Blockvorderseite (102) durchgängige Apertur (109) und ein in der Apertur (109) angeordnetes sowie im Wesentlichen anteroposterior längserstrecktes Ausrichtelement (110) aufweist, welches zum Ausrichten entlang der Whiteside-Linie (W) des Femurs (F) eingerichtet ist.

8. Chirurgisches Instrument (1) nach Anspruch 7, **dadurch gekennzeichnet, dass** das Ausrichtelement (110) relativ zu dem Referenzblock (100) beweglich ist zwischen einer ersten Stellung, in welcher das Ausrichtelement (110) in einem ersten Winkel zu der virtuellen Abstandslinie (L) der Referenzbohrungen (107, 108) orientiert ist, und einer zweiten Stellung, in welcher das Ausrichtelement (110) in einem zweiten Winkel zu der virtuellen Abstandslinie (L) der Referenzbohrungen (107, 108) ausgerichtet ist.

9. Chirurgisches Instrument (1) nach Anspruch 8, **dadurch gekennzeichnet, dass** das Ausrichtelement (110) ein posteriores Ende, welches an dem Referenzblock (100) um eine proximodistal orientierte Schwenkachse beweglich gelagert ist, und ein anteriores Ende (111) aufweist, welches über die Blockoberseite (103) ragt und zur manuellen Bewegung des Ausrichtelements (110) zwischen der ersten Stellung und der zweiten Stellung eingerichtet ist.

## Claims

1. Surgical instrument (1) for use in a knee joint replacement operation, having
a reference block (100) with a proximally oriented block rear face (101) which is configured to bear on a distal end face (S) of a resected femur (F),
a stylus (200) which is arranged on the reference block (100) in the region of an anterior block upper face (103) thereof and has a stylus tip (201) which protrudes proximally beyond the block rear face (101) and is configured to bear on an anterior face (A) of the femur,
two reference feet (300, 400) which are spaced apart mediolaterally from one another and are each arranged on the reference block (100) in the region of a underside (104) thereof lying posteriorally opposite the block upper face (103) and each have an anteriorally oriented foot upper face (301, 401) protruding proximally beyond the block rear face (101), wherein the foot upper faces (301, 401) are arranged in a common reference plane (R) and are each configured to bear on a posterior condyle (KL, KM) of the femur (F),
at least two reference bores (107, 108) which each extend orthogonally to the block rear face (101) continuously from the latter as far as a distally opposite block front face (103) and are each configured for receiving a reference pin which can be introduced into the distal end face (S) of the femur (F), wherein the at least two reference bores (107, 108) are spaced apart from one another along a virtual spacing line (L) which is elongate mediolaterally and parallel to the reference plane (R),
wherein there is at least one compensation element (500) which is configured for form-fitting and/or force-fitting releasable connection to one of the two reference feet (300, 400), and wherein the at least one compensation element (500) has an anteriorally oriented element upper face (501) which comes to bear, instead of the relevant foot upper face (301, 401), against the relevant posterior condyle (KL, KM), **characterized in that** the at least one compensation element (500) has, on its element underside (502), a receiving pocket (503) which is sunk anteriorally and is open posteriorally and in which the reference foot (300, 400) can be received in a form-fitting manner.

2. Surgical instrument (1) according to Claim 1, **characterized in that** the at least one compensation element (500) has, on its element underside (502), an outer rim (504) which protrudes posteriorally, and in particular surrounds the receiving pocket (503), and is connectable in a form-fitting manner to an outer circumference of the reference foot (300, 400).

3. Surgical instrument (1) according to Claim 2, **characterized in that** the outer rim (504) is elastically flexible.

4. Surgical instrument (1) according to one of the preceding claims, **characterized in that** the at least one compensation element (500) has a lateral element section (505), a medial element section (506), a separating gap (507), which separates the two element sections (505, 506) from one another and is elongate proximodistally and is open at one end, and a joint section (508) which is arranged at the other end of the separating gap (507) and connects the two element sections (505, 506) to one another in an elastically movable-joint manner.

5. Surgical instrument (1) according to one of the preceding claims, **characterized in that** there are a plurality of different compensation elements (500, 500', 500''), the different compensation elements (500, 500', 500'') differing, preferably exclusively, in respect of a respective anteroposterior thickness (t1, t2).

6. Surgical instrument (1) according to Claim 5, **characterized in that** there are at least three different compensation elements (500, 500', 500'') having a respective thickness of 1 mm, 2 mm and 3 mm.

7. Surgical instrument (1) according to one of the preceding claims, **characterized in that** the reference block (100) has an aperture (109) reaching between the block rear face (101) and the block front face (102), and an alignment element (110) which is arranged in the aperture (109), is elongate substantially in anteroposterior direction and is configured for alignment along the Whiteside's line (W) of the femur (F).

8. Surgical instrument (1) according to Claim 7, **characterized in that** the alignment element (110) is movable relative to the reference block (100) between a first position, in which the alignment element (110) is oriented at a first angle with respect to the virtual spacing line (L) of the reference bores (107, 108), and a second position, in which the alignment element (110) is oriented at a second angle with respect to the virtual spacing line (L) of the reference bores (107, 108).

9. Surgical instrument (1) according to Claim 8, **characterized in that** the alignment element (110) has a posterior end, which is mounted on the reference block (100) so as to be movable about a proximodistally oriented pivot axis, and an anterior end (111), which protrudes beyond the block upper face (103) and is configured for manual movement of the alignment element (110) between the first position and the second position.

## Revendications

1. Instrument chirurgical (1) destiné à être utilisé dans une opération de remplacement de l'articulation du genou, présentant
un bloc de référence (100) avec un côté arrière de bloc (101) orienté de manière proximale, qui est adapté pour s'appuyer sur une face frontale distale (S) d'un fémur réséqué (F),
un palpeur (200) qui est agencé sur le bloc de référence (100) dans la zone d'un côté supérieur de bloc antérieur (103) de celui-ci et qui présente une pointe de palpeur (201) qui dépasse de manière proximale du côté arrière de bloc (101) et qui est adaptée pour s'appuyer sur une face antérieure (A) du fémur,
deux pieds de référence (300, 400) espacés médiolatéralement l'un de l'autre, qui sont agencés respectivement sur le bloc de référence (100) dans la zone d'un côté inférieur de bloc (104) de celui-ci, opposé postérieurement au côté supérieur de bloc (103), et qui présentent respectivement un côté supérieur de pied (301, 401) orienté antérieurement et dépassant proximalement du côté arrière de bloc (101), les côtés supérieurs de pied (301, 401) étant agencés dans un plan de référence commun (R) et étant adaptés chacun pour s'appuyer sur un condyle postérieur (KL, KM) du fémur (F),
au moins deux alésages de référence (107, 108), qui s'étendent respectivement orthogonalement au côté arrière de bloc (101) de manière continue de celui-ci jusqu'à un côté avant de bloc (103) distalement opposé et qui sont respectivement adaptés pour recevoir une broche de référence pouvant être introduite dans la face frontale distale (S) du fémur (F), les au moins deux alésages de référence (107, 108) étant espacés l'un de l'autre le long d'une ligne d'espacement virtuelle (L), qui s'étend longitudinalement de manière médiolatérale et parallèlement au plan de référence (R),
au moins un élément de compensation (500) étant présent et étant adapté pour une liaison amovible par complémentarité de forme et/ou par force avec l'un des deux pieds de référence (300, 400), et l'au moins un élément de compensation (500) présentant un côté supérieur d'élément (501) orienté antérieurement, qui est adapté pour s'appuyer sur le condyle postérieur (KL, KM) concerné à la place du côté supérieur de pied (301, 401) concerné,
**caractérisé en ce que** l'au moins un élément de compensation (500) présente sur son côté inférieur d'élément (502) une poche de réception (503) enfoncée antérieurement et ouverte postérieurement, dans laquelle le pied de référence (300, 400) peut être reçu par complémentarité de forme.

2. Instrument chirurgical (1) selon la revendication 1, **caractérisé en ce que** l'au moins un élément de compensation (500) présente sur son côté inférieur d'élément (502) un bord extérieur (504) faisant saillie postérieurement, entourant notamment la poche de réception (503), qui peut être relié par complémentarité de forme à une périphérie extérieure du pied de référence (300, 400).

3. Instrument chirurgical (1) selon la revendication 2, **caractérisé en ce que** le bord extérieur (504) est élastiquement flexible.

4. Instrument chirurgical (1) selon l'une quelconque des revendications précédentes, **caractérisé en ce que** l'au moins un élément de compensation (500) présente une section d'élément latérale (505), une section d'élément médiale (506), une fente de séparation (507) qui sépare les deux sections d'élément (505, 506) l'une de l'autre et s'étend longitudinalement de manière proximodistale et est ouverte à une extrémité, et une section d'articulation (508) qui est agencée à l'autre extrémité de la fente de séparation (507) et relie les deux sections d'élément (505, 506) l'une à l'autre de manière élastique et articulée.

5. Instrument chirurgical (1) selon l'une quelconque des revendications précédentes, **caractérisé en ce que** plusieurs éléments de compensation différents (500, 500', 500'') sont présents, les différents éléments de compensation (500, 500', 500") différant, de préférence exclusivement, en termes d'une épaisseur antéropostérieure respective (t1, t2).

6. Instrument chirurgical (1) selon la revendication 5, **caractérisé en ce qu'**au moins trois éléments de compensation différents (500, 500', 500") sont présents, ayant une épaisseur respective de 1 mm, 2 mm et 3 mm.

7. Instrument chirurgical (1) selon l'une quelconque des revendications précédentes, **caractérisé en ce que** le bloc de référence (100) présente une ouverture (109) continue entre le côté arrière de bloc (101) et le côté avant de bloc (102), et un élément d'alignement (110) agencé dans l'ouverture (109) et s'étendant longitudinalement de manière essentiellement antéropostérieure, qui est adapté pour s'aligner le long de la ligne de Whiteside (W) du fémur (F).

8. Instrument chirurgical (1) selon la revendication 7, **caractérisé en ce que** l'élément d'alignement (110) est mobile par rapport au bloc de référence (100) entre une première position dans laquelle l'élément d'alignement (110) est orienté selon un premier angle par rapport à la ligne d'espacement virtuelle (L) des alésages de référence (107, 108) et une deuxième position dans laquelle l'élément d'alignement (110) est orienté selon un deuxième angle par rapport à la ligne d'espacement virtuelle (L) des alésages de référence (107, 108).

9. Instrument chirurgical (1) selon la revendication 8, **caractérisé en ce que** l'élément d'alignement (110) présente une extrémité postérieure qui est montée sur le bloc de référence (100) de manière mobile autour d'un axe de pivotement orienté proximodistalement, et une extrémité antérieure (111) qui dépasse au-dessus du côté supérieur de bloc (103) et qui est adaptée pour déplacer manuellement l'élément d'alignement (110) entre la première position et la deuxième position.
